# EUROPEAN PATENT APPLICATION

(11) **EP 3 763 302 A1**
(43) Date of publication of application: **13.01.2021**
(21) Application number: 19185883.6
(22) Date of filing: 11.07.2019
(51) Int. Cl.: A61B 17/16, A61B 17/88, A61B 90/00, A61B 17/00

(54) **ELECTRONIC MODULE FOR A SURGICAL INSTRUMENT**

(71) Applicant: Shamsollahi, Javad, 3007 Bern (CH)
(72) Inventor: Shamsollahi, Javad, 3007 Bern (CH)
(74) Representative: Schneiter, Sorin

(57) **Abstract**

The present invention relates to an electronic module for a surgical tool. The electronic module may be removably attached to the surgical tool or it may be encompassed within a part of the surgical tool. The electronic module is configured to learn about the evolution of measurable parameters which are detectable by at least a part of the tool to which the electronic module is attached or incorporated and, during an operation mode of the tool, to determine optimum values of a parameter depending on the evolution of the parameter or parameters being monitored.

## Description

### Technical Field

The present invention relates generally to the field of surgical instruments and more particularly to means and methods for drilling, or screwing fasteners, into tissue such as bone. The invention also relates to a electronic module for a surgical instrument, to the surgical instrument comprising the module, and to a method of producing an algorithm for the electronic module.

### Background Art and problems Addressed by the Invention

In the field of orthopedics, it is sometimes required to use a screw to fix a broken bone to a plate or more generally to implant a component into a bone by applying torque. Implant components such as orthopedic plates and screws are commonly used for treatment of millions of bone fractures annually. The Association for the Study of Internal Fixation (AO Foundation), a nonprofit organisation founded in 1958 for the improvement of care for people with musculoskeletal injuries, established the basic principles of anatomic reduction, stable fixation, preservation of blood supply and early active mobilisation in the field of fracture treatment.

Internal fixation is a known method for stabilising a bone fracture. The action of the screw thread against the surrounding material plays a main and crucial role in ensuring a successful fixation during the time required for healing of the fracture to complete. The tightening torque of the screws has an impact on the interface of the screw threads and the surrounding material.

Orthopaedic screw insertion procedures therefore require particular attention to be paid to the amount of tightening torque that is used during the screw insertion process. An optimum tightening torque ensures that the screw is adequately tightened to serve purposes of mechanical stability of the fixation. If the tightening torque is too low, then the screw cannot transmit the forces applied. If the tightening torque is too high, then the interface between the screw and the bone is destroyed and purchase is lost. In the case of locking screws, applying overly high torque may result in the interface between the threaded head of the screw and the plate being destroyed. Various factors affecting the optimal torque include the dimensions of the screw, the thread pattern and the material from which the screw is made, as well as the local mechanical resistance of the bone, which may depend on the type of bone, and/or the quality or density of the bone at the place or places where the screw is to be inserted. In the case of locking screws, the optimal torque may further depend on characteristics of the thread on the head of the screw and the corresponding thread on a plate with which the head of the screw engages. Similarly, when drilling into a bone, depending on which type of bone and which part of the bone, there is an optimal torque. In the case of drilling, there may also be an optimum drilling speed.

During orthopaedic interventions in which an implant component, such as a screw, is implanted into tissue such as a bone, adequate tightening torque must be applied to the implant component to provide appropriate tension to reliably hold the component in place without stripping the thread on the component or damaging the bone or tissue into which the component is implanted. Reliable tensioning avoids the need for post-implant operations to retention the implant component. Factors affecting torque include screw geometry and specification, the material used for the screw, local mechanical resistance of the bone, which is related to bone density and thickness and friction between the bone and the thread of the screw. It is also worth noting that cancellous screw insertion torque depends significantly on the bone microarchitecture and bone mineral density.

It is known that bone quality may vary from patient to patient depending on the patient's sex, age, life style, medical history or genetical factors. Bone quality may even vary depending on the anatomical location of a bone in a same patient. Bone density may be measured to provide an indicator of bone quality. Even subtle changes in bone density may lead to large variations in the strength or the elastic modulus of the bone. Furthermore, bones are generally made of different component structures, known as cortical bone and cancellous bone. Cortical bone is the compact part of the bone, generally on the walls of long bones, while cancellous bone is the spongy part of the bone, generally in the inner part of the long bones.

In order to provide a stable fixation after anatomic reduction, orthopaedic plate and screw systems may be used. Known plate/screw systems include those which use locking screws, which feature a threaded head, and those which use non-locking screws, having a non-threaded head. In plate/screw system using locking screws, the locking threaded head of the screw engages and locks into the threaded plate hole. Such locking screws both the screw head and the bore in the plate are threaded and the head of the screw locks generally comprise mechanical torque limiters/attachments, whose purpose is to limit the tightening torque of the screw threads onto the plate. Such torque limiters are convenient for the plate/screw system using locking screws because both the screw head and the plate are made of metal, which is a solid, homogeneous and consistent material.

On the other hand, most known non-locking screws are inserted using conventional, manually-operated screwdrivers. Determination of an appropriate endpoint for the tightening of the screw is therefore left to the surgeon's own judgment. Consequently, the tightening torque of the screw shaft thread against the bone depends on the subjectivity of an individual surgeon and may even depend on the strength which a particular surgeon is capable of exerting. Surgeons having different numbers of years of experience or of different gender or age, or having different manual strengths, apply their own judgement as to the endpoint for the tightening of the screw. Therefore, the chances of having large variations between the applied toques for same conditions between different surgeons is high. Furthermore, it is worth bearing in mind that the optimum screw insertion torque into cancellous bone depends greatly on the bone microarchitecture and bone mineral density.

It is known from work presented by Goheen et al. that there is wide variation in the ability of clinicians to judge or to otherwise apply adequate insertion torque to implant components. According to Goheen et al., the importance of being able to apply a torque consistently and optimally cannot be overstated. Care must be taken to ensure that components are not overtightened or under tightened, so the appropriate preload of implant components is achieved. For proper implant torque to be applied during implant of components it would therefore be desirable and advantageous to have a suitably calibrated torque device.

Surgical instruments which comprise a programmable torque limiting device are known. During operation, the surgeon sets a maximum torque value and the instrument either warns the surgeon when the maximum torque value is reached or automatically stops the application of any further torque once the maximum torque has been reached. However, since the optimum insertion torque and tightening torque varies greatly from patient to patient, depending on which bone or part of a bone is to be operated on, and further depending on the bone quality and screw specification, such instruments are not ideal especially for the non-locking screws.

The Skills Lab booklet, published by the AO Foundation, which is the largest global organisation for training orthopaedic surgeons, discloses that if torque for bone screws is too high, the interface between screw and bone is destroyed and purchase is lost, while if the torque is too low, the screw cannot transmit the forces applied. The booklet goes on to recommend that surgeons practice in order to become proficient at applying the optimal torque during operations. Practice involves performing screw implant operations in practice structures representing different bone qualities, such as cadaver bones or model bones, to gain the feel for when the optional torque is achieved. The practice also involves gaining a feel for over-tightening and under-tightening of screws. The booklet discloses that the optimal torque lies between 60-85% of the maximum torque, which is usually defined as being a torque which would strip the thread.

A. Feroz et al. inserted two hundred screws into cadaver bones and found that 21% of the screws became stripped or ran the risk of becoming stripped. This led to a recommendation of optimum screw tightening being one which provides a tightening torque of between 65% and 89% of the stripping torque, which is a similar value to that published by The AO Foundation.

Using non-invasive three-dimensional micro-computed tomography techniques, J. Steiner et al. presented one of the first studies to localise and quantify peri-implant bone damage along the entire length of the screw. It is claimed that this technique could be a promising method to investigate more systematically the effect of peri-implant bone damage on primary implant stability, such as damage caused by excessive loading and screw insertion.

Majewicz et al. designed and used a one-degree-of-freedom friction-based haptic simulator to present bone screw insertion scenarios to expert surgeon users. Even though it is claimed that the haptic simulator can be used for virtual reality training of the surgeons to practice applying an optimal tightening for orthopedic screws, it does not represent exactly the measured resistance of the bone and has limited ability to generate sufficient torque to reach the peak seen in the normal bone real data. Furthermore, this device cannot be used intraoperatively and therefore the trained users might lose the gained practice of applying optimal torques of different screws in different bones after some time.

Several surgical technique guides prepared by certain orthopaedic manufacturers provide warnings on overtightening of small non locking screws and recommend using the "two finger tightening" method in order not to overtighten the screws. The "two finger tightening" method involves holding the screwdriver handle using two fingers instead of using the entire hand thereby preventing overtightening of the screws.

Wilkofsky et al. quantified the torque produced by a number of different surgeons using the "two finger tightening" method for inserting screws. The more experienced surgeons applied statistically greater torque producing greater forces than the younger, less experienced surgeons. It is thought that the more experienced surgeons knew how close they could get to a "two finger tightness" level without actually stripping the screws. It was also shown that among surgeons there is a wide variability in torques applied. This may be associated with variations in the desired compression between bone fragments or the possibility of screws being stripped, especially in osteoporotic bone.

It would therefore be desirable to provide surgeons with a more reliable means for applying optimal tightening torque during osteoporotic implant operations. It would also be advantageous to be able to monitor, or otherwise control, the applied insertion torque during the operation, particularly for non-locking screws and specifically for the cancellous screws which are mainly engaged in the spongy part of the bone, where the application of the optimal torque is critical to the success of the fixation. Such monitoring and controlling of the applied torque would also be beneficial for avoiding screw heads becoming cut off during removal, recess stripping and controlling the absolute applied torque between the screw head and plate. It would also be advantageous to control the process of drilling into tissue such as bone.

US 2014/0222012 discloses a programmable screw driver and a method for affixing screws into bone. The programmable screwdriver includes a torque sensor for measuring torsional input during screw insertion, a rotational motion sensor for measuring the rotation of the screw driver, and a microprocessor. The torque sensor measures the torque exerted during screw insertion and sends this information to the microprocessor, which compares the measured torque to a pre-determined torque level. When the pre-determined torque level is achieved the microprocessor relies on measurements from the rotational sensor to provide a warning when a subsequent pre-determined rotational limit has been reached. The microprocessor relies on pre-determined torque and rotational limits without taking account of actual operational conditions such as the quality of the bone being operated upon.

US 2013/0338669 and US 2008/0300510 disclose a tool specifically adapted for determining the local mechanical resistance of a porous body, in particular bone. From a torque value measured with the instrument, one can draw conclusions about the suitability of the bone to implant a hip screw or hip plate. These teachings thus propose a separate instrument for making an advance measurement for assessing physical properties on bone. This instrument is not helpful for determining an optimum torque to be applied for a particular patient.

EP 2832285 describes a surgical drill or saw for determining the local mechanical resistance of a bone by determining the volume or removed material and the applied specific energy used to remove the material.

US 2005/0131415 discloses motorized drill and screwdriver comprising sensors for detecting torque and rotational angle, and a microcontroller for processing the sensed parameters and storing the processed results.

The present invention addresses the problems depicted above.

### Summary of the Invention

In an aspect, the invention provides an electronic module comprising one or more sensor configured to detect at least one parameter related to one or more physical properties or condition of a patient's tissue and to convert the detected parameter to an electrical signal, the electronic module further comprising a battery provided to supply electric energy to said sensor.

In an aspect, the invention provides an assembly comprising the electronic module.

In an aspect, the invention provides a control system for a hand-held surgical instrument comprising a handle and an effector part, which is configured to act on a patient's tissue or on a piece to be connected to a patient's tissue, said the control system comprising: the electronic module of the invention, a computer readable code configured to determine a relevant information that is relevant to a surgeon using the surgical instrument in said operational environment.

In an embodiment, said computer readable code is further configured to perform an action when said relevant information is determined, wherein said action is selected from one or both of: (a) induce the output of information via an output-unit, and (b) acting on said surgical instrument so as to affect the continued use of the surgical instrument in said operational environment.

In an aspect, the invention provides a module preferably connectable to (and detachable from) conventional orthopedic instruments such as screwdrivers and power tools for producing data which can be collecting the data (physical properties or condition of a patient's tissue) for a postoperative studies and evidences.

In an aspect, the invention provides a method of collecting data, the method comprising storing on a database the data generated by the one or more sensors of the electronic module of the invention when using a surgical instrument comprising the electronic module, preferably together with data related to the patient on which the surgical instrument was used.

In an aspect, the invention provides the use of the electronic module of the invention for collecting data, in particular data related to a patient.

Further aspects and preferred embodiments of the invention are defined herein below and in the appended claims.

Further features and advantages of the invention will become apparent to the skilled person from the description of the preferred embodiments given below. These embodiments are provided by way of examples and the present invention is not intended to be limited to the particular embodiments as illustrated in the figures.

### Brief Description of the Drawings

**Figure 1** schematically illustrates a surgical instrument in accordance with an embodiment of the invention.
**Figure 2A** schematically illustrates the electronic module according to an embodiment of the invention for use with a surgical instrument.
**Figure 2B** schematically illustrates the electronic module according to another embodiment of the invention for use with a surgical instrument.
**Figure 3** shows an exemplary surgical instrument comprising a electronic module in accordance with an embodiment of the invention.
**Figure 4** shows an exemplary surgical instrument and a display for displaying an output control signal in accordance with an embodiment of the invention.
**Figure 5A** illustrates a curve established by the control system in accordance with an embodiment of the invention.
**Figure 5B** illustrates the logic of the algorithm used by the control system in accordance with an embodiment of the invention.
**Figure 6** illustrates a curve established by the control system in accordance with another embodiment of the invention.
**Figure 7** illustrates the method of operation of the surgical instrument in accordance with an embodiment of the invention.

### Detailed Description of the Preferred Embodiments

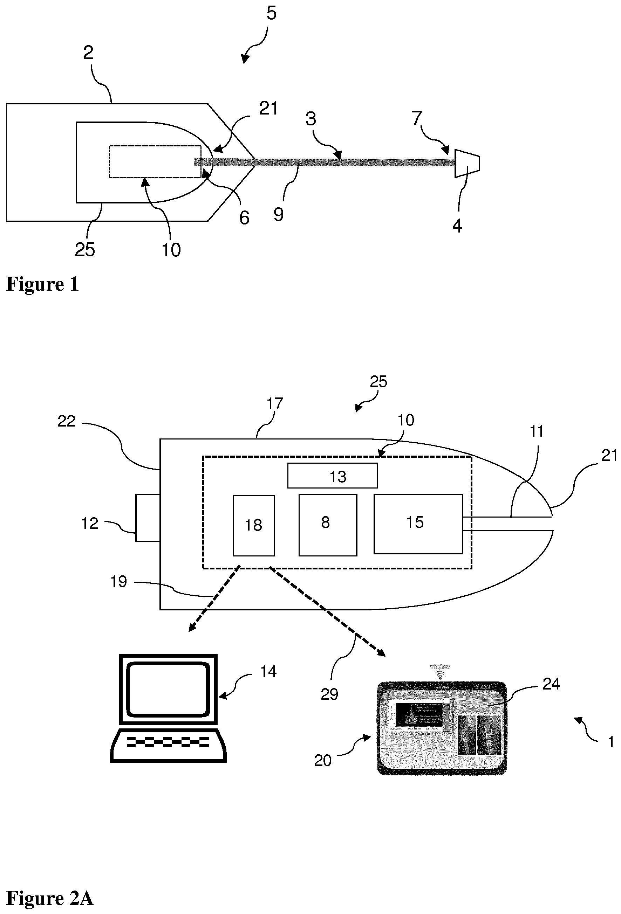

**Figure 1** shows a hand-held surgical instrument **5** comprising an electronic module **10** according to an embodiment of the present invention. The surgical instrument **5** comprises a handle **2,** for manipulation of the instrument by a surgeon, and an effector part **3.** The effector part **3** is the part that directly interacts with, comes in contact with and/or acts on a patient's tissue or on a piece to be connected to a patient's tissue,

Preferably, the surgical instrument is a hand-held surgical instrument. The surgical instrument **5** may be a screwdriver, a drilling instrument, and a sawing instrument, for example. The surgical instrument may be motorized or hand-driven. In an embodiment, the surgical instrument is hand-driven and not motorized.

The effector part **3** depends on the surgical instrument or, inversely said, defines the surgical instrument. The effector part **3** may be selected from a screwdriver shaft, a drill bit, a guide wire and a Kirschner wire (K-wire), for example. Accordingly, if the instrument **5** is a screwdriver, the effector part is preferably a screwdriver shaft. If the instrument is a drilling instrument, the effector part may be selected from a drill bit, a guide wire and a K-wire, for example. In an embodiment, the effector part **3** is an existing product, such as a commercially available screwdriver shaft, drill bit, guide wire, etc.

In the embodiment shown, the elongate effector part **3** has an elongate shaft **9** and proximal and distal ends **6, 7,** respectively.

At the distal end **7,** the effector part preferably has a head **4** adapted to perform a particular operation depending on the type of surgical instrument. For example, the head **4** may be a screwdriver head for screwing a screw into a bone of a patient. In other examples the head **4** is the extremity or tip of a drill bit or of a guide or of a K-wire.

At the proximal end **6,** the effector part **3** is inserted or otherwise connected to or in contact with the first end **21** of an electronic module **10.** The expression "electronic module" is used in this specification to generally refer to an entity or assembly comprising several electronic components. It is noted that the electronic module is preferably comprised in or part of an assembly **25** comprising also mechanical components.

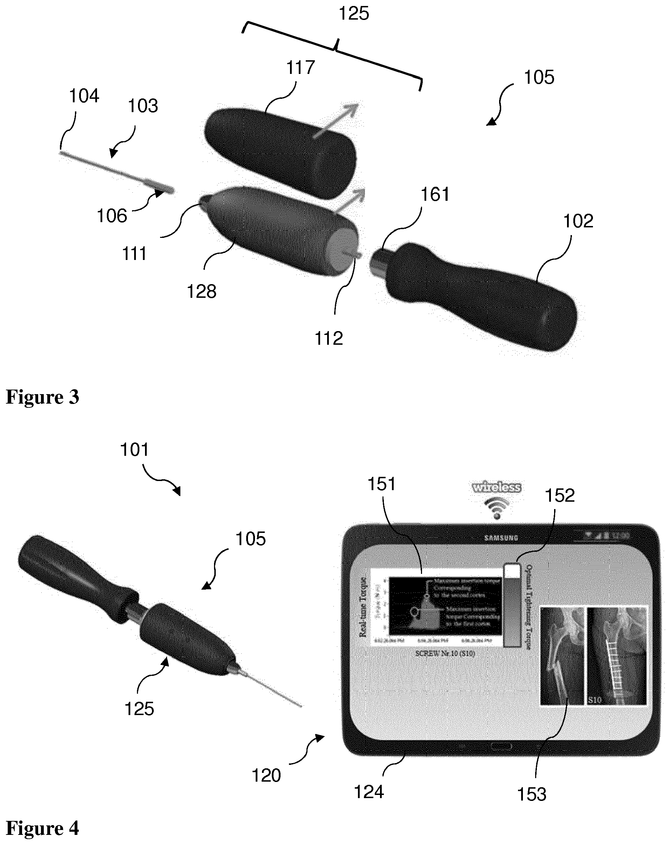

In an embodiment, as illustrated in Fig. 1, the electronic module **10** is housed in the handle **2** of the surgical instrument **5.** In other embodiments, as shown for example in Fig. 3, the electronic module **10** may be provided outside the handle, for example at an extremity of the handle. The electronic module **10** may be totally or partially be housed in the handle or may be outside and/or separate from the handle **2.**

**Figure 2A** shows the assembly **25** comprising the electronic module **10** as well as the electronic components **8, 15, 18, 13** of the module **10** according to a first embodiment. The proximal end **6** of the effector part **3** (Fig. 1) is adapted for being connected with a connector **11** of the assembly **25.** The assembly **25** and in particular the electronic module **10** are arranged in such a manner that when the effector part **3** is connected to the assembly **25,** a sensor **15** of the module is capable of detecting a parameter related to an operational environment in which at least the head **4** of the effector part **3** of the surgical tool **5** evolves and to convert the detected parameter to an electrical signal. For example, the proximal end **6** of the effector part **3** is in contact with the sensor or the connector **11** of the module and transmits the parameter from the effector part **3** to the sensor **15.**

In accordance with the invention, the surgical instrument **5** is configured to act on a patient's tissue or on a piece to be connected to a patient's tissue. Preferably, the sensor **15** is configured to detect at least one parameter related to one or more physical properties of a patient's tissue and to convert the detected parameter to an electrical signal.

For example, if the surgical tool **5** is a screwdriver, then the head **4** of the effector part **3** is a screwdriver head and an environment in which the head of the screwdriver evolves during a surgical operation for implanting a screw is the screw head. The sensor **15** may be a sensor configured to produce a signal corresponding to the torsional force (torque) exerted by the screwdriver and in particular by the screwdriver head **4** on the head of the bone screw. This torsional force is a parameter that is related to one or more physical properties of a patient's tissue, in particular to the properties of the bone into which the bone screw is inserted. Generally, if the bone tissue has higher density or bone quality, the torsional force that needs to be applied will be higher.

In an embodiment, said parameter detected by said sensor **15** is selected from the group consisting of: reaction torque, angle of rotation, apical force, apical pressure, axial displacement and temperature. Of course, the electronic module may comprise more than one sensor and may thus be suitable a combination of several of the mentioned parameters. Also sensors capable of detecting more than one parameter are envisaged. In a preferred embodiment, said parameter is reaction torque.

In an embodiment, said parameter is determined as a function of time, distance and/or angle of rotation, for example. The electronic module **10** may comprise further sensors as appropriate, for example for detecting angle of rotation, distance, and the like.

The electronic module may comprise one or more sensors of different types including one or more sensors selected from the group consisting of: torque sensors, force sensors configured to detect an axial force on the effector part, rotational angle sensors configured to detect an angle of rotation of the blade, rotation speed sensors configured to detect a rotational speed of the effector part, insertion depth sensors configured to detect an axial displacement of the effector part and temperature sensors configured to detect a temperature at the environment of operation of the instrument.

As further shown in Fig. 2A the electronic module also comprises a battery **13** and a transmitter **18** for wirelessly transmitting and/or broadcasting data **19, 29.** The battery **13** provides the electric energy for allowing the other electronic components (e.g. **15, 8, 18**) to operate, preferably as described in this specification.

In an embodiment, the electronic module **10** further comprises an amplifier **8** configured to amplify one or more signals generated by said one or more sensor **15.** The amplifier **8** thus preferably generates amplified signals. In an embodiment, the transmitter **18** is configured to directly transmit and/or broadcast the amplified signals **19, 29** generated by the amplifier. Accordingly, in an embodiment, the data **19, 29** transmitted by said transmitter **18** are said amplified signals.

As further shown in Fig. 2A, the electronic module preferably comprises a housing **17.** The housing **17** preferably protects the electronic components (e.g. **13, 15, 8, 18**) of the module **10.** The electronic components may be provided on a support **128** (Fig. 3) comprising, for example, a printed circuit board, on which the electronic components are provided.

In an embodiment, the electronic module comprises a second connector **12,** preferably provided at a second end **22** of the assembly **25.** As will be described in more detail with reference to Figs 3 and 4, the second connector is preferably provided in cases where the electronic module is not housed inside the handle, but is releasably connected to the handle.

In an embodiment, the system of the invention preferably comprises a data processing entity **20.** In an embodiment, the data processing entity **20** comprises a receiver configured to receive the wirelessly transmitted data transmitted by said transmitter **18,** to run said computer readable code to determine said relevant information, and to perform said action, such as a display of information on said output unit **24.**

Figure 2A shows two data processing entities **14** and **20,** which comprise a receiver and/or a wireless communication module configured to receive the data **19** and **29** transmitted by the transmitter **18.**

The data processing entity **14** is not necessarily part of the control system **1.** As will described further below, the data processing entity **14** is preferably used to store data and to establish an algorithm suitable to be used in the system of the invention.

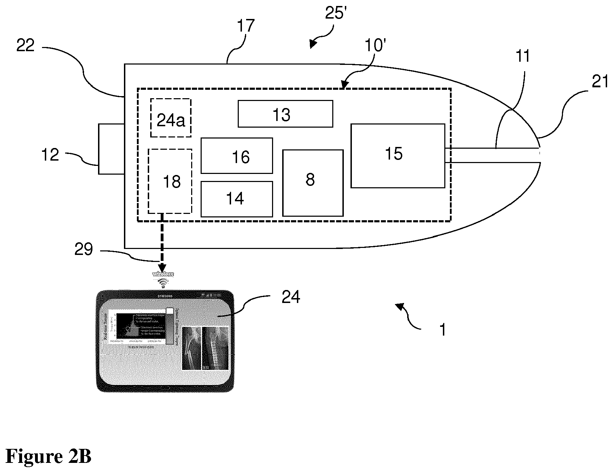

In principle, the data processing entity **20** is not limited to a particular form. In an embodiment, the data processing entity **20** is selected from the group consisting of a computer, a desktop computer, laptop computer, computer tablet and a smartphone. In Figs 2A, 2B and 4 the data processing entity **20, 120** is shown to be a computer tablet.

The control system **1** preferably comprises a data processing entity **20,** which comprises and/or runs a computer readable code that is configured to determine an information that is relevant to a surgeon using the surgical instrument **5** in the operational environment, in particular during a surgical intervention in which said surgical instrument **5** is used. In this specification, this information is also referred to as "relevant information".

The expression "computer readable code" encompasses software and/or firm-ware in low-level and/or high-level computer languages, including computer readable instructions in the form of machine code.

Preferably, the relevant information is in relation to the parameter detected by said sensor. Furthermore, the relevant information is preferably related to one or more physical properties of a patient's tissue and the relevancy applies preferably to the operational environment in which the surgical instrument is used.

Preferably, the relevant information is also in relation to an undesirable and/or even dangerous situation, which may be associated with a risk for the health of the patient, such as that the determination of the relevant information generally aims at preventing the undesirable event from happening and/or at reducing the risk for the health of the patient.

In an embodiment, said computer readable code comprises and/or makes use of an algorithm for determining said relevant information.

In an embodiment, said computer readable code and in particular said algorithm, is configured to determine and/or predict the relevant information in real time, for example while receiving said data emitted by said transmitter **18.**

In an embodiment, said relevant information is selected from the group consisting of: (a) the detection of the occurrence of an event, (b) the determination of a relevant value or relevant value range, in particular with respect to said parameter. Preferably, said event is an event that is occurring while the surgical instrument is being in an operational environment, during a surgical intervention.

For example, said relevant value and/or relevant value range may be a relevant information, in as far trespassing of said relevant value for said parameter may be associated with an undesirable situation.

In an embodiment, said event is selected from the group consisting of:
(i) the detection of a situation where, during a surgical intervention, a relevant value and/or relevant value range of said parameter has been reached;
(ii) the occurrence of a contact of said surgical instrument or of said piece to be connected to a patient's tissue with tissue having different properties and/or a different structure.

Regarding point (ii), this may encompass one or more selected from the group of: 1) the contact of a head (4) of the effector part (3) or of a tip of said a piece to be connected to a patient's tissue with a second or far cortex with, 2) the passing of the head (4) or of said tip of said piece into the far cortex, and 3), the breaking of the head (4) or of said tip of said piece through the far cortex.

Further regarding point (ii), this may more generally be described as the detection of said head (4) or said bone screw tip approaching, getting into vicinity or getting in contact with of said a second or far cortex. The transition between the cancellous bone and cortical bone may be a more or less smooth, continuous transition. The detection of one or more of points 1) - 3) above generally aims at detecting a situation before said head (4) has passed entirely through said far cortical bone, such that the computer readable code can perform the appropriate action before said head has passed through said far cortex.

The events the occurrence of which is detected by the algorithm will be discussed elsewhere in this specification.

Preferably, said computer readable code is further configured to perform an action when said relevant information is determined, wherein said action is selected from one or both of: (a) induce the output of information via an output-unit **24,** and (b) acting on said surgical instrument **5** so as to affect the continued use of the surgical instrument **5** in said operational environment.

The action performed by the computer readable code may involve the display of information and/or an action affecting the continued use of the surgical instrument **5** is an on-going surgical intervention. In this latter case, the action performed by the computer code is generally a remedial action.

If the computer readable code is configured to perform a remedial action, the action generally depends on the surgical instrument. For example, if the surgical instrument is a drill, remedial action may encompass one or more selected from the group consisting of reducing the drill rotation speed, reducing the axial force on the drill bit or stopping the drill altogether, for example. If the surgical instrument is a screwdriver, the remedial action may comprise uncoupling the handle from the effector part (screwdriver shaft) so as to actively prevent further tightening.

If the action concerns the output of information, the information is preferably communicated via an output unit **24.** The output unit may have any suitable form and may be comprise for example, one or more selected from the group of a loudspeaker, a light emitting entity such as an LED, a screen, touch screen, display, monitor, and the like.

Accordingly, the output generated by the computer readable code may be an acoustic output (e.g. loudspeaker) and or a visible signal (e.g. LED, screen).

The output unit **24** is preferably comprised in the system **1** of the invention, and may be part of the data processing entity **20.**

The output as produced by the output unit **24** is preferably related to the relevant information. If the output unit **24** is a screen as shown in Figs 2A, 2B and 4, the output may be a number (numerical indication), a graph, a bar graph, a color, one or more displayed words (literal indication of information) and so forth. If the output unit is a source of light, the output may simply consist in switching on the source of light. If the output unit is or comprises a loudspeaker, the output may comprise a tone or sound, an alarm sound and/or pronounced words, for example.

Displays are preferred output units, because they allow continuously showing information and may thus show the evolution of a parameter in the course of s surgical intervention, for example. For example, a display may show a graph and or a bar and may provide a continuous indication of the relative or absolute amount of a relevant parameter. Furthermore, a display is advantageous as it may use color as an indicator of the relevancy or urgency of the event and/or displayed information. In case of a display, relative or distinctive levels may be indicated, for example corresponding to "too low", "optimal" and "too high".

In an embodiment, the output unit **24** is the screen of said data processing entity **20,** such as said desktop computer, laptop computer, computer tablet and a smartphone.

In a preferred embodiment, the output of information via an output-unit **24** generally involves a warning or alert, for example in order to inform the surgeon about the occurrence of a potentially undesirable event and/or the imminence of such an undesirable event if the surgical instrument is further used. The warning may tell the surgeon to stop continuing in the same manner and/or to change continuing with the intervention in the same manner, for example.

For example, if the parameter by said sensor is reaction torque, the relevant information is related to reaction torque. The display may directly show the reaction torque, for example numerically or graphically, and/or using color as indicating safe values of reaction torque.

As mentioned above, the relevant information determined by the computer readable code may involve the (a) detection of the occurrence of an event and/or (b) the determination of a relevant value or relevant value range, in particular with respect to said parameter.

Regarding (a), this encompasses that a value or value range as determined under (b) actually corresponds to an occurring event under (a). Regarding point (b), this may involve the determination of a relevant value or value range in advance, that is, the value or value range is determined in real time during a surgical intervention before an event corresponding to the value is occurring.

Preferably said relevant value range represents a range in which the relevant value for the detected parameter is supposed to lie or indeed lies. Of course, the narrower the range, the closer the determined parameter will be to the relevant value of the parameter.

In an embodiment, the relevant value is an optimum value and the relevant value range is an optimum value range, that is a range in which the optimum value lies.

For illustrating an embodiment, it is supposed that the surgical instrument is a screwdriver and that parameter related to one or more physical properties of a patient's tissue is reaction torqued determined when screwing a bone screw into bone tissue of the patient. In this case, there is an optimum torque value or value range at which the screw insertion is such that the screw retention in bone tissue is at a maximum. The optimum torque value is reached after the screw head has reached the surface of the bone or of the plate to be connected to the bone by the screw. At this moment, the reaction torque increases sharply. At some moment, further application of torque via the screwdriver may entail damage to the bone tissue and stripping of bone tissue by the screw thread. This is undesirable, since at this moment, the retention force that the bone may provide to retain the screw starts to diminish rapidly. Generally, the optimum torque is reached before stripping of bone tissue.

In the example above, the relevant value for the detected parameter or the relevant value range for the detected parameter is the optimum torque value for fixing the bone screw in bone tissue of the particular patent and/or a range in which the optimum torque value is supposed to lie.

In this example, if the optimum torque value is reached during an ongoing surgical intervention, the system may induce an output which comprises a warning, which may involve the display of information in any manner in order to inform the surgeon of the fact that the optimum value has been reached (or will soon be reached), and that further tightening of the screw should be stopped or stopped soon.

In the embodiment shown, in Fig. 2A, the computer readable code and/or algorithm are comprised in the data processing entity **20.** Thanks to this, the electronic module comprises relatively few components and is thus comparatively inexpensive. This is an advantage in embodiments where the assembly **25** comprising the electronic module is or comprises a disposable, for example a single-use entity. While the screen **24** is integrated as part of the data processing entity in Fig. 2A, it is noted that the output unit **24** may be a separate device, for example a screen that is separate from the data processing entity.

Buttons for operating the electronic module, possibly start and reset buttons, the configuration of the module, and the like, may be provided on the data processing entity **20,** for example activatable via a graphical user interface (GUI).

In an embodiment, the electronic module **10** lacks a microcontroller, microprocessor and/or any data processing entity. In an embodiment, the electronic module **10** lacks a memory, for example lacks random access memory (RAM) and/or lacks read-only memory ROM. As mentioned with respect to embodiment shown in Fig. 2A, the "intelligence" of the system resides in the data processing entity, which is preferably physically separate from the surgical instrument.

**Figure 2B** shows another embodiment of the system **1** of the present invention. In this embodiment, the electronic module **10'** comprises further electronic components, such as a microprocessor or microcontroller **14** and, optionally, a memory **16.** A memory may be comprised on the microcontroller **14** and a separate memory **16** may thus not be necessary. The memory may be RAM, ROM and/or both.

In this embodiment, the computer code and/or algorithm that is capable of determining said relevant information, is comprised and/or run by the electronic module **10'.** The electronic module **10'** may also comprise an optional output unit **24a,** such as a screen, light source, or loudspeaker, for outputting said output produced by the microprocessor and/or computer readable code.

Preferably, the embodiment shown in Fig. 2B still comprises a transmitter **18** for wirelessly transmitting data. In this embodiment, the data **29** may not necessarily be the amplified signals, but may be the relevant information, such as calculated value of the parameter, or may be directly the information to be displayed on the screen **24.**

In another embodiment, the transmitter **18** in Fig. 2B also transmits said signal produced by the sensor, preferably as amplified by amplifier **8.**

A separate screen **24** for displaying the data and/or information **29** transmitted wirelessly may still be present, although it is no longer mandatory if output unit **24a,** on the module **10'** is present.

Not shown in Figs 2A and 2B are start or reset buttons or input buttons or entities, with which the operation of the electronic module **10, 10'** can be started, reset and/or configured. In a preferred embodiment, starting, measuring and/or choosing a configuration of the electronic module is accomplished via the data processing entity **20** and via wireless data transmission from the data processing entity **20** to the electronic module **10, 10',** if an external data processing entity is present in the system.

**Figure 3** shows a surgical instrument **105** comprising an electronic module according to a preferred embodiment of the invention. In Fig. 3, the electronic components are not visible, as they are hidden by the mechanical components of the assembly **125.** The electronic module is comprised inside the assembly **125.** In terms of electronic components, the electronic module of assembly **125** may correspond to any one of the electronic modules **10** or **10'** as shown in Figs 2A and 2B, respectively, for example.

In an embodiment, said electronic module is configured to be replaceably and/or detachably interconnected between said handle and said effector part.

As shown in Fig. 3, the assembly **125** comprising the electronic module is an intermediate and/or interconnected device, that can be connected on the one hand to the handle **102** of a conventional surgical tool, such as a conventional surgical screwdriver having a handle **102** and to a removable effector part, which here is a screwdriver shaft **103.** Preferably, the assembly **125** is comprises connectors **111, 112,** that allow it to be used with existing surgical instruments.

In an embodiment, the assembly **125** is removably and/or detachably connected to said surgical instrument **101,** preferably between said handle **102** and said effector part **103,** and preferably outside said handle.

In an embodiment, the assembly **125** comprises a first connector **111** for connecting the proximal end **106** of the effector part **103** with said electronic module and/or with said assembly **125** and a second connector **112** for connecting the handle with said assembly **125.**

The handle **102** preferably comprises a connector **161** at one of its end, generally referred to as the distal end of the handle **102.** The connector **161** is preferably configured to match a connector provided at the proximal end **106** of the effector part **103,** as is conventional. Indeed, the effector part **103** may be a replaceable and/or detachable piece, which can be selected by a surgeon from a series of end effectors having the same connector **106** but a different head **104,** depending on the specific operation a surgeon intends to perform. For example, if the surgical instrument is a screwdriver, the effector part **103** is selected such that the screwdriver head **104** matches the screw head of the bone screw that is to be screwed. As another example, if the effector part **103** were a drill bit, the surgeon may typically select the effector part depending on the diameter of the hole to be drilled, for example. Surgical instruments comprising different, interchangeable effector parts, such as a series of different screwdriver shafts are already known and in use. It is an advantage of the present embodiment that it can be used with pre-existing surgical instruments by being interconnectable between the handle and the effector part of conventional surgical instruments.

The connector **161** of the handle **102** is preferably outside the handle or, if it is inside the handle, it is in the distal end of the handle, such that the assembly **125** will be connected outside the handle **102** and is thus a separate and removable entity of the surgical instrument **105** as a whole, comprising the electronic module **10, 10'.**

In an embodiment, the assembly **125, 25** comprising the electronic module **10, 10'** is removably and/or detachably connected in said surgical instrument **101.**

In an embodiment, the control system comprises an assembly **25, 25', 125** comprising: said electronic module **10, 10'** a housing **17, 117,** first and second connectors **11, 12; 111, 112** for connecting said assembly to said handle **2, 102** and to said effector part **3, 103** of said surgical instrument **5, 105,** respectively. The assembly **25, 25', 125** preferably comprises first and second ends **21, 22** as described above with the assembly **25, 25'** shown in Figs 2A and 2B.

A first connector **111** is provided at the first end, and a second connector **112** is provided at the second end of the assembly **125.** The first connector **111** is configured to be connected to the proximal end **106** of the effector part **103,** such as a conventional screwdriver bit or drill bit. The second connector **112** is configured to be connected to the connector **161** of the handle **102.**

The first connector **111** of the electronic module may have a mechanical connection similar to the connector **116** of the handle, and the second connector **112** of the electronic module may have a mechanical connection similar to the proximal end **106** of the effector part **103,** such that the assembly **125** can be interconnected between the end effector and handle of a typical or conventional surgical instrument having a removable end effector part, such as a removable screwdriver bit or removable drill bit.

The connectors **111** and **112** may be of any suitable connector, coupling, chuck or adaptor type, and may be selected, for example, from the group consisting of a dental coupling, Stryker coupling, hexagonal coupling, AO coupling, TPS coupling, 1/4" Square lock ("Quick Lock®"), 3/4" Square lock ("Quick Lock®"), Tri-Flat or Tri-Lobe, Small Hudson, Large Hudson, Jacob-Hall (Zimmer-Hall). Various different connectors are marketed, for example, by the company Tecomet (www.tecomet.com) in Kenosha, WI 53144, USA. A preferred coupling is the standard AO quick connector type, which are commonly used on conventional instruments in the domain.

In an embodiment, the first connector **111** is a female part configured to receive the proximal end **106** of an elongate effector part, such as a screwdriver shaft, and the second connector **112** is configured as a male part, resembling or identical to the proximal end of the effector part, configured to be received by the connector **161** of the handle **102.**

The assembly **125** may comprise or be equipped with a removable and possibly disposable housing **117.** Thanks to the housing, the electronic module and/or the support **128** on or in which the electronic components are fixed as such may be re-usable, with the same surgical instrument **5, 105** or with a different one, for example. For example, after use in an operational environment, the housing **117** may be removed, and a new, preferably sterile housing may be used with the electronic module **110.**

As already described above, the assembly **125** comprises, amongst other the electronic components as shown in Figs 2A and 2B. As can be seen in Fig. 3, the assembly preferably comprises said detachable housing **117,** and a support or chassis **128,** on which the first and second connectors **111, 112** are fixed. The electronic components are preferably fixed and or housed in said support **128.** In the embodiment shown, the housing **117** is removably connected with said support **128.** The housing **117** may be disposable, and/or may be autoclavable and reusable, such that the support may be reused while the housing is removed for autoclavation and/or replaced by a new housing after a surgical intervention.

In the embodiment shown in Fig. 3, the housing, cover or capsule **117** is configured to cover the module to avoid problems related to contamination. The electronic module could also be reusable, but is generally not autoclavable. The rest of the reusable parts could be cleaned and sterilized according to the normal hospital procedure, after the electronic components have been removed.

In an embodiment, the electronic module is a replaceable consumable or is comprised in an assembly **25, 125** that is a replaceable consumable.

In some embodiments, the assembly **125** is sterile and disposable as a whole, including the housing **117,** the support **128,** the connectors, **111, 112,** and the electronic components. In this case, a detachable housing **117** may not be required.

In another embodiment, the housing **17, 117** is disposable and/or for single use, but the support **128,** the connectors **111, 112,** and the electronic module **10, 10'** are for multiple use. In this case, a user may simply replace the housing and continue using the assembly. Since the assembly **25, 125** is covered by the disposable cover **17, 117,** the assembly **25, 125** does not need to be autoclaved and can be reused with a new or autoclaved cover **17, 117.**

In yet another embodiment, the housing **117** and the support **128** are re-usable and/or autoclavable, but only the electronic module **10, 10'** is for single use. In this case, only the electronic module needs to be replaced and is preferably used for a single surgical intervention only and/or with one patient only during a surgical intervention.

Detachable parts, such as the assembly **25, 125,** the housing **17, 117,** and so forth, are preferably detachable manually, such that a specific instrument for detaching the unit is not required.

Preferably, the assembly **25, 125,** comprising the electronic module, is configured for being manually connected with the surgical instrument **5, 105,** without requiring a specifically created and/or a standard tool.

Thanks to the features disclosed above, the electronic module of the invention may be used in combination with conventional and/or pre-existing surgical instruments, including instruments that were not initially conceived to be used with the electronic module of the invention. Furthermore, the assembly or one or several parts thereof (the support, the electronic module, etc), can be conceived as a disposable unit. It is noted that conventional surgical instruments frequently have removable end effector parts, for example in order to allow separate sterilization, or in order to allow the use of a single handle in combination with different effector parts, such as with screwdriver bits adapted to different screw heads and the like.

**Figure 4** shows a control system comprising the assembly **125** integrated in the surgical instrument **105** of Fig. 3 and a data processing entity **120** comprising an output device **124,** such as a display or screen. As mentioned with respect to Figs 2A and 2B, the electronic module **10, 10',** comprises preferably a transmitter, transceiver and/or wireless communication module **18.** The transmitter **18** may transmit signals which have been generated by the sensor **15** and which have preferably been amplified (Fig. 2A), or the transmitter may directly transmit relevant information, such as a determined optimum value, for example (Fig. 2B).

In Fig. 4, the data processing entity is a computer tablet **120** with screen **124.** The tablet **120** runs a computer readable code, which is configured to determine a relevant value for the parameter determined by the sensor, or a relevant value range, or another relevant information.

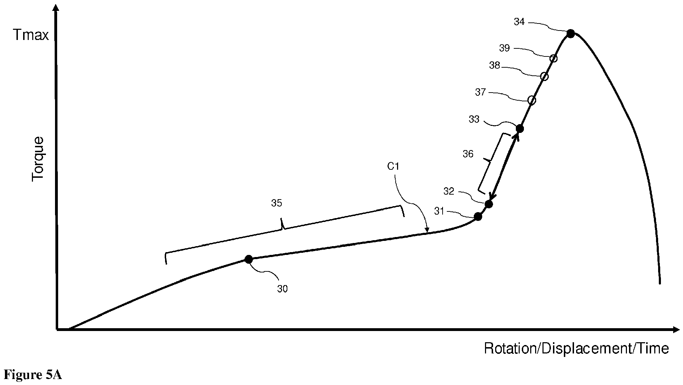

In the embodiment shown in Fig. 4, the display **124** displays a graph **151** with a curve and/or a bar plot **152** on which the value of the parameter determined by the sensor **15** is visualized. The graph **151** may show torque as a function of time, rotation or displacement, for example as described in more detail with respect to Fig. 5A.

The display **124** may display further information, such as images **153** related to the patient, preferably to the patient's body part or an illustration of the anatomy that is involved in the surgical intervention. These images may originate from a camera and may thus reflect in real time information at the patient's tissue during the intervention. The system of the invention may thus be conceived to represent information as mentioned above on a pre-existing display used for displaying information related to a surgical intervention, for example. This may be achieved, for example, by simply installing the computer readable code on a data processing entity that is already used by a surgeon and/or that is already present in a surgery room.

In an embodiment, said tissue is bone tissue.

In an embodiment where said tissue is bone tissue, said surgical instrument may be a screwdriver and said parameter is reaction torque. The reaction torque may be determined as a function of the angle of rotation, time, and/or distance. Preferably, the reaction torque is determined as a function of the angle of rotation. In this case, the control module preferably comprises a further sensor or entity for detecting the angle of rotation.

In an embodiment where said tissue is bone tissue, said surgical instrument is a drill. In this case, the preferred parameter may be reaction torque and/or applied axial force. These parameter or parameters may be determined as a function of the angle of rotation, time, and/or distance.

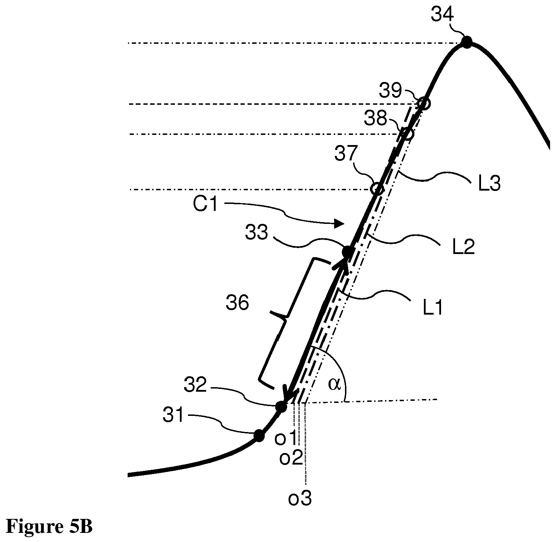

**Figures 5A** and **5B** illustrate an embodiment of the invention, in particular related to the concept of the computer readable code and the algorithm used by the system to determine in real time the information that is relevant to an on-going surgical intervention in which the surgical instrument comprising the electronic module is used.

In an embodiment, said tissue is a bone tissue of a patient, said surgical tool **5** is a screwdriver acting on a bone screw, wherein said detected parameter is related to reaction torque, and wherein the control system (1) is configured to determine in real time an optimal torque and/or optimal torque range.

Fig. 5A shows an example of the evolution of torque values detected by a torque sensor configured to detect reaction torque exerted on a surgical screwdriver during an operation of screw insertion into a bone. The vertical axis of the curve may thus be the torque value, usually expressed in newton-metres (N.m). The value of torque evolves during the operation of screw insertion. Depending on the units of the horizontal axis, the evolution may be expressed in terms of time or rotation angle, number of turns or insertion depth, for example. It is noted that the curve of Fig. 5A is somewhat smooth. If torque is determined as a function of time, the curve **C1** may have steep rises and falls along the curve where the twisting motion of the screwdriver starts and stops. The steep rises and falls may be eliminated by signal processing while the curve is being established. Furthermore, when the torque is determined as a function of rotation angle or displacement, the steep rises and falls are preferably absent from the beginning, as the curve only takes into account torque when the screw rotates and advances in the bone tissue. In general, the invention encompassing smoothing the curve as much as possible without losing valuable data. Signal processing may take place in the external data processing system **20, 120** (Fig. 2A). In other embodiments, signal processing is conducted in the electronic module **10'** (Fig. 2B).

It is noted that the shape of the curve **C1** depends on the one hand on the nature, quality and/or properties of the tissue, here the bone tissue.

On the other hand, the shape of the curve depends, of course, on other factors, linked to the surgical instrument and, if a bone screw is to be inserted, on the screw. For example, the screw specification (such as dimension and material of the screw, the screw thread pitch, whether or not the screw is going directly into bone or into a plate, whether the screw is non-locking or locking).

Yet other factors that determine the shape of the curve are whether or not the screw is inserted into a pre-drilled hole (which is generally the case), and the conditions of the predrilled hole (actual diameter, straightness etc.), the angle of engagement into the bone, and possibly temperature.

These other factors, which are thus not related to the tissue characteristics, may be referred to herein as "known" or "technical factors". These technical factors will not be discussed in much further detail here, as they are preferably taken into account in any suitable way. For example, the system of the invention preferably comprises an input entity or device, where the technical factors that are known to the surgeon, such as the screw type, predrilled hole condition, and the like, are input. The system of the invention may thus duly take these technical factors into account. In other embodiments, the system of the invention lacks an input device and is configured to determine the relevant information in the absence of further input. Also encompassed is the situation where some of the "technical factors" are input and others are determined by the system.

We will further discuss here those factors that may not be known in advance or not precisely, and which thus cannot be fed into the system in advance of a surgical intervention. These factors are basically those that are related to the characteristics of the tissue, for example the bone tissue, and may therefore be referred to as "tissue-related factors". The tissue-related factors are thus preferably individual characteristics, depending within certain limits on a particular patient.

It is noted that in the prior art, devices have been disclosed which typically encompass determining in advance characteristics of an individual bone tissue, for example, US2008/0338669, US 2008/0300510. In these teachings, a separate surgical tool is used in a preceding intervention before the real surgical intervention with curative purpose is performed, so as to determine, in advance, bone characteristics. In the real surgical intervention (e.g. insertion of the screw), the information gathered during the preceding intervention can then be used. In contrast, the system of the present invention preferably does without a separate instrument and in particular without a preceding determination step, since the characteristics of the tissue are preferably determined in real time during the real surgical intervention.

Depending on the operation and the variables and factors mentioned above, the curves representing the evolution of torque values in a situation as described above generally have certain landmarks in common. For example, at the first part of the curve **35** may the torque may increase gradually as the screw enters the cortical bone and reach a small peak or inflection **30,** as it enters the cancellous bone (in case of a monocortical engagement, for example). Then, as the head of the screw begins to make contact with the bone surface or with the plate to be fixed by the screw there is another inflection **31,** this time in a direction that is the opposite to the inflection **30.** Inflection **31** is characterized by a sharp increase of the torque value. The slope of the upwardly rising curve may pass through landmark point **32,** from which on the increase of torque is linear. The linear region **36** is generally representative of the elastic deformation of the bone tissue. There is an inflection **33** when the linear region ends. The point **33** is a landmark point indicating the end of the linear part of the curve. A further landmark point **34** is at the peak of the curve, when torque is reduced abruptly as stripping of the screw occurs because the tissue has been deformed (destroyed) to such an extent that there is no grip of the screw in the tissue any more. The peak **34** represents the maximum torque (Tmax) that could be measured with a particular bone tissue and further depending on the technical factors mentioned.

Referring to the curve **C1** in Fig. 5, it is noted that the exact form of the curve, the positions of the landmark points **30-34** and regions **35, 36** depend, in absolute terms and in relation to each other, on said "technical factors", which are generally known to the system, and on said "tissue-related factors".

If a maximum stripping torque value (Tmax) of a bone tissue is known or predictable (peak **34**), it is possible to determine the optimum torque value or optimum value range from that maximum value. According to literature, the optimum torque value is in the range of about 60-85% of Tmax. In Fig. 5A, points **37, 38,** and **39** represent 60%, 70% and 85% of Tmax. Accordingly, points **37** and **39** represent the end points of an optimum torque range and point **38** represents an exemplary optimum torque value.

In an embodiment, said computer readable code comprises and/or makes use of an algorithm for determining said relevant information, wherein said algorithm configured to use successive and/or incremental electrical signals when the surgical instrument is used in an operational environment in order to determine one or more landmark points **30-33** or regions **35, 36** in a curve based on said successive signals.

In an embodiment, said computer readable code determines said relevant information as it occurs. This embodiment corresponds to the detection of the occurrence of an event, for example the detection of a situation where, during a surgical intervention, a relevant value and/or value range of said parameter has been reached.

Taking the example of reaction torque value or range, the algorithm may determine in real time that, during an on-going surgical intervention, an optimum reaction torque has been reached, such as point **38** in curve **C1.** The system may then produce an appropriate output in the moment where the optimum value has been reached.

In an embodiment, the relevant information is determined in an anticipatory manner, for example in the anticipation of a particular event that is going to occur while the surgical instrument is being in an operational environment, during a surgical intervention. This embodiment, encompasses, for example, the determination of a relevant value or relevant value range, in particular with respect to said parameter, preferably before said relevant value or value range is reached during an on-going surgical intervention. Turning to the example described with reference to Fig. 5B, an optimum toque value (or optimum range) may be determined before the optimum torque value (or optimum range) is reached during an on-going surgical intervention.

Accordingly, in an embodiment, said computer readable code comprises and/or makes use of an algorithm that determines said relevant information in advance, that is before the event that corresponds to the relevant information occurs in an on-going surgical intervention. In other words, the system, and in particular said computer readable code, anticipates the occurrence of a particular event and produces an output, for example to inform the surgeon that a particular event is going to occur soon, or any other output as disclosed in this specification.

In an embodiment, said system **1** is configured to determine said relevant information from said landmark points **30-33** and/or landmark regions **35, 36** and/or from an extrapolated curve determined from said landmark points and/or landmark regions.

Embodiments where the system is configured to determine an extrapolated curve may be used where the system determines the occurrence of relevant information in advance, that is before the occurrence of the event that corresponds to the relevant information. For illustrating this point, the algorithm may be configured to anticipate the entire curve as shown in Fig. 5A in the course of a surgical intervention once the points **30-32** have been passed and/or once the linear phase **36** has been reached, or the end thereof at point **33.**

Thanks to determining the curve by anticipation from landmarks points during an on-going intervention, the system may determine the relevant values or value range **37-39** for the parameter determined by the sensor **15,** thus as optimum reaction torque. As mentioned above, optimum torque can be determined as a percentage of Tmax, wherein said percentage has been previously reported in the literature (see above), or can be determined by the manufacturer of the system.

Based on the fact that the shape of curves for particular surgical intervention have an overall similar shape when performed with different individual and further based on the detection of characteristic positions in the curve (said landmark point and regions) by a suitable algorithm, the system can determine when a particular point or range in the curve is reached, such as the optimum torque value **38,** a start point **37** or end point **39** of an optimum value range.

In this manner, the algorithm may determine a relevant information, which may be a relevant value or value range, in particular with respect to said parameter. This determination preferably occurs in the course of an on-going surgical intervention and may occurs in advance, that is before the relevant (or optimum) value or range has been attained.

In summary, the system of the invention may detect an event that constitutes a relevant information as the event occurs, for example when an optimum torque is reached or when contact with the far cortex is detected. In some embodiments, the system anticipates the occurrence of an event, such that the relevant information is the detection of an event before it occurs. This may be done by anticipating the values of certain landmark points and/or relevant values during the earlier stages of an on-going surgical intervention.

Since the characteristics of the curve depend on "tissue-related factors", which may not be known by the system beforehand, the algorithm preferably comprises instructions allowing an interpretation or analysis of the tissue in real time, during an on-going surgical intervention using the instrument **5, 105,** so as to be able to determine said relevant information.

The algorithm that determines said relevant information in real time is preferably based on data gathered previously in experimental settings. An exemplary way of how to create the algorithm will be discussed further below in more detail. For the time being it is sufficient to know that data collected from experimental settings, and possibly also from learning in real operational settings is used for establishing, testing and validating the algorithm. Hereinafter, some general principles of exemplary algorithms will be discussed.

In an embodiment, the algorithm comprises a categorization of tissues in types or groups, such that a determination of relevant information, such as a relevant value, etc, is made on the basis of the group in which a tissue and/or patient is categorized in real-time during a surgical intervention. In this case, the relevant information, such as an optimum value, may ensue directly from said category.

In an embodiment, the algorithm is configured to attribute, in real time, a given tissue upon which said surgical instruments acts, to a particular category out of a plurality of categories. Preferably, the system determines the relevant information based on the category with which a tissue is associated in real time in the operational environment, for example.

The categories used by the algorithm may be qualitative or may be established arbitrarily. For example, if the categories are qualitative, they may relate to tissue quality or density, for example bone quality, bone density, and so forth. Taking bone quality as an example, the categories may reach from low bone quality to high quality, or may simple express bone quality with a number in a given range, e.g. bone quality 1 to bone quality 10.

In an embodiment, an optimal value or optimal value range is associated with a particular category, such that said optimal value or optimal value range ensues directly from said category. During the use of the surgical instrument with a patient's tissue, the algorithm may be configured to associate the tissue to a particular quality category and to deduct the relevant value and/or value range from that quality category.

In other embodiments, the algorithm does not use a categorization of tissues and simply determines a relevant value and/or value range (or event) directly and specifically for a given tissue in real time. In this case, the relevant value and/or value range may not be selected from a number of predetermined relevant values associated with a particular category.

Fig. 5B illustrates the principles of an algorithm in which a relevant information, in particular the optimum torque and/or an optimum torque range is determined based on theoretical knowledge of mechanical properties as well as learned predetermined curve landmarks. In Fig. 5B, an extract of the curve **C1** of Fig. 5A is shown, showing in greater detail the part of the curve starting shortly before inflection **31,** when reaction torque raises sharply, as the head of the bone-screw comes to lie on the outer bone surface. This part of the curve covers also the linear region **36,** the end of the linear region and the part up to the turning point **34** at Tmax.

The algorithm creates three auxiliary and/or imaginary straight lines **L1, L2** and **L3,** which start at the height of the start **32** of the linear region **36** of the curve **C1,** but which are offset on the x-axis compared to the linear region. The straight lines **L1, L2, L3** are offset by increasing values **o1, o2** and **o2,** respectively, from the point **32.** In the example shown in Fig. 5B, the lines **L1, L2, L3** are parallel to the linear region **36** of the curve, such that they will cut the curve **C1** at some length after the linear region has ended curve and the starts to bend after point **33** to head towards turning point **34.** The points where the lines **L1, L2, L3** cut the curve **C1** correspond to points **37, 38, 39,** which define the optimum torque value **38** or the range (**37-39**) in which the optimum torque lies **37-39.**

As illustrated in Fig. 5B, in an embodiment, the computer readable code comprises instructions to create one or more auxiliary line and/or function, and determining an intersection of said auxiliary line and/or function with a curve representing the evolution of the parameter as detected by said sensor, wherein said intersection constitutes said relevant information. As mentioned, said evolution of said parameter may refer to one or more selected from evolution of the parameter in dependence of rotation, displacement and time.

If the curve **C1** is a curve that has been extrapolated based on landmarks occurring earlier in the curve and detected in real time, then said intersections and thus said points **37-39,** which represent said relevant information, can be determined in advance.

Turning to the particular example of an algorithm determining the optimum value of reaction torque when fixing a bone screw, the curve corresponds to measured reaction torque established as a function of time, displacement or angle of rotation, for example. The point **38** where the auxiliary line **L2** intersects with the curve corresponds to the optimum torque value as determined by the system. Therefore, once the algorithm detects that an evolving curve established in real time during the insertion of the bone screw intersects with said auxiliary line **L2,** the system produces an output in order to inform the surgeon that the optimum torque value has been reached.

As shown also in Fig. 5B, a plurality of auxiliary lines may be created by the algorithm, for example two lines **L1, L3,** to determine an optimum value range for the parameter determined by the sensor, defined by part of the curve **C1** lying between the intersections **37, 39,** of the curve with each of the two lines **L1, L3,** respectively.

As becomes apparent, one, several or all of the intersections **37, 38, 39,** in Fig. 5B represent the relevant information determined by the algorithm. The positions of these intersections depend on the shape of the curve **C1** on the one hand and on the characteristics of the auxiliary lines **L1, L2, L3,** on the other hand. In particular, the lengths of the offsets **o1-o3** determine the point of the intersections. Another determinant is the orientation of the lines **L1-L3.**

In an embodiment, said curve **C1** comprises a linear region **36,** and wherein said auxiliary line is positioned so as to be in parallel to said linear region or at an angle with respect to said linear region.

In an embodiment, the one or more auxiliary line **L1, L2, L3,** is parallel to the linear region **36** of the curve **C1.** In this case, the angle α, which represents the angle of the linear region **36** with respect to horizontal, also defines the orientation of the one or more auxiliary line.

In another embodiment, the one or more auxiliary line **L1, L2, L3,** is skewed or at an angle with respect to the linear region **36** of the curve **C1.** In this case, the angle between the auxiliary line and horizontal is different from α. For example, the angle may be larger than α, such that the intersection happens earlier than and/or before the situation in Fig. 5B where the angle of the auxiliary line is α. According to another example, the angle between the auxiliary line and horizontal is smaller than α, such that the intersection happens later than and/or after the situation where the angle of the auxiliary line is α.

In an embodiment, the offset and/or the orientation of the one or more auxiliary line **L1, L2, L3,** is determined by the algorithm, preferably based on the values of the parameter detected by the sensor in real-time, during a surgical intervention.

In an embodiment, if said one or more lines **L1, L2, L3,** are not parallel to said linear region **36,** the algorithm may determine the angle of said one or more lines with respect to horizontal and/or with respect to said linear region **36,** preferably based on the values of the parameter detected by the sensor in real-time, during a surgical intervention.

For example, the positions of landmark points or regions earlier in the procedure (e.g. **30-32, 35**), may be used by the algorithm to establish particular vales for any one or all of **o1-o3,** and/or for the angle of the line with respect to horizontal. Alternatively, or in addition, the algorithm may use the angle α of the linear region with respect to horizontal for determining the offsets and/or orientations of the auxiliary lines.

The algorithm may also change the particulars of the auxiliary line as the intervention is on-going and as further measurements are made by the sensor. For example, if the slope α of the linear region **36** is to be taken into account, it is necessary that the intervention of screw tightening, for example, proceeds into the linear region before the definitive position of the auxiliary line is established.

In the embodiment discussed above, the auxiliary lines **L1, L2, L3** are preferably straight lines. In other embodiments, the invention encompasses that the algorithm establishes an auxiliary function that may not correspond to a straight line, and to detect the intersection of the auxiliary function with the curve **C1** so as to determine a point representing a relevant information or part thereof.

Fig. 5B also illustrates the determination of a relevant value range, defined by the presence of two auxiliary lines **L1, L3,** with the cure **C1.** The determination of a value range, or even of just the point **37** defining the start of the range **37-39,** can be considered as the anticipation of an event in advance, i.e. before it occurs, such as the anticipation of the event at point **38,** when the optimum torque value is reached. Referring to the example discussed above, when during an on-going surgical intervention, the system detects that point **37** is reached, the computer readable code may perform an action, such as the generation of a first warning, informing that the optimum value range will soon be reached. Therefore, the determination of a value range and the detection of the occurrence the start of the range is reached may be considered as an anticipation of the event.

While Figs 5A and 5B illustrate curves in which the sensors **15** used to detect said parameter is a torque sensor, such curves may be created in analog manner with other physical parameters related to one or more physical properties or condition of a patient's tissue. In an embodiment, said sensor is a temperature sensor and the system of the invention is configured to take account of the value of the temperature during the operation and to perform an action when a certain temperature threshold has been reached in order to avoid an undesirable and/or dangerous situation during an operation, such as burning of bone tissue (cell necrosis), especially a drilling operation. Said action may include displaying information such as for how long a temperature has remained above a pre-determined threshold, and/or displaying a warning. Said action may include in addition and/or instead of displaying information, remedial action, such as reducing the rotation speed of a drill bit, for example.

In an embodiment, said relevant information aims at warning a surgeon before the head of the surgical instrument, or the tip of a piece to be connected to bone tissue, such as a bone screw, passes through the far or second cortex.

During drilling a hole or screwing a bone screw into bone tissue, there is a risk that the drill bit, Kirschner/guide wire or bone screw, respectively, passes unnoticed through the bone and exits the bone, for example on the side opposite the side of insertion. This situation is known as "plunging" in the field of surgery. Once this happens, there is a risk that soft tissue lying behind the bone is damaged.

In this case, the relevant information may also be considered as an event, namely the occurrence of contact with, passing through or breaking through the far cortex.

The computer readable code and/or algorithm of the system of the invention is preferably configured for detecting one or more selected from the group selected from: (a) the contact of the tip of a drill bit or bone screw with the far cortex, (b) the passing of the tip of the drill bit or bone screw through the far cortex, and (c) the breaking of the tip of a drill bit or bone screw through the far cortex.

The cortical bone forms the denser and generally harder shell of bones, which surrounds the spongy bone in the inside the bone. The different parts of the bone have different physical properties, which is why the control module of the invention can be used for detecting the contact and/or interaction of a bone screw or drill bit with the different types of bone tissue.

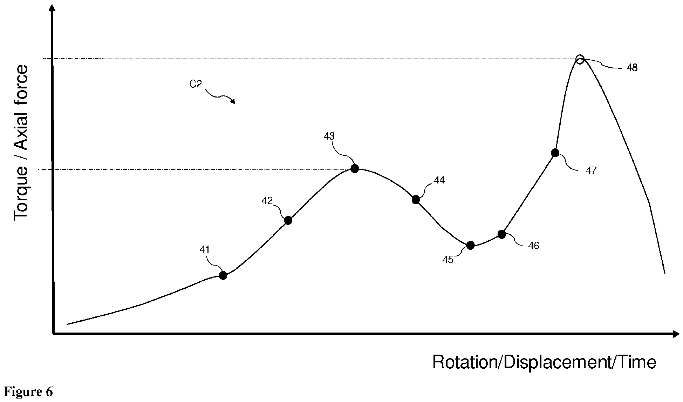

**Figure 6** illustrates the detection of the second cortical bone. As described with respect to Fig. 5A, the graph shows the values of a physical parameter as determined by the one or more sensor **15.** These values may be the results of successive measurements and the curve **C2** may be smoothed by signal processing software and/or by a data processing entity, as necessary.

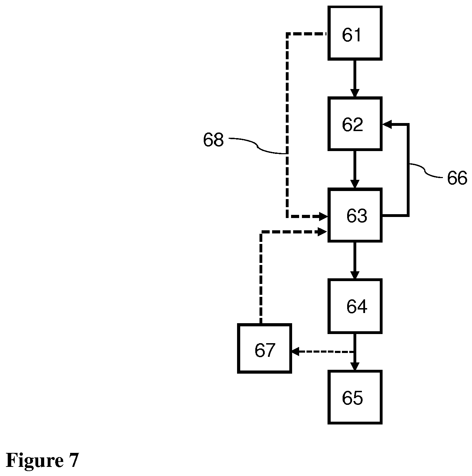

The exemplary curve **C2** in Fig. 6 is established in the course of a drilling operation where a drill bit passes through the near cortex of the bone at peak **43,** then through the cancellous bone and finally through the far or second cortex at point **48.** Similarly, this type of curve **C2** can be representative of inserting a screw through the first cortex, then through the cancellous bone and finally through the far cortex. Fig. 7 illustrates the evolution of torque (N.m) monitored by the control module during the operation using the surgical instrument.

The landmarks **41, 42, 43, 44, 45, 46, 47** which may be seen on this curve and recognized by the algorithm include entry to the first, or near cortex **41,** an inflection or peak **43** where passage from the near cortex to the cancellous bone occurs, another inflection **45,** in the opposite direction, where the passage from the cancellous bone to the second, or far, cortex is reached, then a second peak inflection **48** where the far cortex is traversed. The curve **C2** may also comprise particular regions, such as linear regions and the like, and some or all of the landmarks may be the same or similar to those in the curve **C1** shown in Fig. 5A, since in both cases a bladed shaft, such as a screw or a drill bit is driven into bone tissue.

The computer readable code and/or the algorithm of the present invention may be configured to detect the various phases of the operation as the different regions of the bone are reached and signal, either to the user or to the instrument, when the second cortex is reached or passed through.

As mentioned earlier, in a preferred embodiment, the algorithm is preferably configured to determine said relevant information *in advance.* Preferably, the algorithm determines that a particular event is close, that is, happening within a predetermined interval of the independent variable on the abscissa (angle of rotation, displacement, time). The algorithm may use the landmark points to create an extrapolation of the curve so as to determine the position of peak **48,** for example, before this point is reached during an ongoing surgical intervention.

In the situation where a drill bit passes through a bone it is advantageous to detect the event such as breaking through the far cortex in advance, due to the speed of the drill compared to the human reaction time. In an embodiment, the system of the invention preferably produces an output that results in a remedial action such as discussed above, for example stopping the motor of a motorized drill or reducing drill speed, such that the complete transversal through the bone by the drill is effectively prevented.

The invention is preferably not limited to a particular way by which the algorithm detects the relevant information or event in advance.

Herein above the "technical information" has been defined as information related to a particular surgical instrument that is used, bone screw specification, and the type of surgical intervention (e.g. drilling or screwing) which are typically known by the user in advance any which may been feed into the system by a suitable input.

In an embodiment, the system is configured to detect one or more of said technical information. In an embodiment, the system is configured which type of operation is being performed based on one or more parameters such as torque, the "shape" of the torque evolution (to determine whether the tool is being manually operated or operated by a motor), the speed of rotation and so on. Accordingly, the computer readable code may make use or comprise a further algorithm, which detects the type of intervention that is conducted. This further algorithm may then select amongst several algorithms the very algorithm to be used for the type of surgical intervention that was detected. The very algorithm may then determine the relevant information, such as torque value or range limits, speed limits or temperature limits, for example, for the particular surgical intervention.

In an embodiment, the algorithm is configured to detect whether a drilling operation or a screwing operation is being conducted.

The algorithm used by or comprised in the computer readable code is preferably established on the bases of measurements made in an experimental setting on artificial tissue, cadaver tissue, animal tissue, or any suitable model of the tissue in question. It is noted that the algorithm may be further improved by data collected from a real operational environment.

For the purpose of creating the algorithm, the surgical instrument comprising the electronic module may be considered to be used in a "learning mode" or "learning phase". The learning mode may be illustrated with reference to **Fig. 2A****.**

When using the surgical instrument on sample tissue in an experimental setting (eg. on artificial tissue), the data **19** transmitted by transmitter **18** are received by a data processing entity **14,** which generally also comprises a memory for storing the data.

After having collected a large number of data, an algorithm is preferably prepared or selected which is the best in predicting and/or detecting the relevant information. When working with sample data in an experimental setting, the surgical instrument may be used until the end of a particular curve. For example, a bone screw may be tightened beyond the point of maximum torque, and a drill may be used to pierce a bone entirely, passing also through the far cortex. Thanks to this data, it is possible to determine said relevant information, such as the optimum torque retrospectively for a given sample, for example as a given percentage in case of optimum tightening torque (e.g. 60-85% of maximum torque). For example, during the learning phase, the entire curve as shown in Fig. 5A is established for characterizing a given tissue. In the particular example of tightening torque, literature knowledge may be taken into account for determining the optimum value of the parameter.

The learning mode thus differs from a real surgical intervention in that during the real surgical intervention, it is generally a goal to prevent certain events and/or points of the complete curve, such as to prevent, for example, the screw to go beyond too far beyond Tmax (point **34**) in Fig. 5A.

In other embodiments, the relevant information is determined by further tests, for example by biomechanical testing, for example by checking the solidity of a screw tightened using a presumptive optimum torque or using an arbitrarily selected value of said parameter. The algorithm preferably take into account the results obtained by specific biomechanical testing and is validated using this results.

In some embodiments, the invention relates to a method for producing an algorithm for a control module and/or for the surgical instrument of the invention.

The method comprises:
a) providing a surgical instrument comprising the control module **10, 10'** of the invention,
b) using the instrument to act on a sample tissue or on a piece, such as a screw, to be connected to said sample tissue,
c) detecting and storing signals produced by the sensor **15,** while the instrument is being used in the preceding step, said sensor **15** being configured to detect at least one parameter related to one or more physical properties of a said tissue,
d) generating a curve based on the signals measured in step c),
e) repeating steps b)-d) with different tissues and/or artificial tissues,
f) using one or more learning models for predicting and/or determining a relevant information (37-39) of said parameter,
g) using said learning model as said algorithm.

In an embodiment, the method further comprises: (f.1) providing a plurality of different learning models and for predicting and/or determining an relevant value or relevant value range (37-39) of said parameter, and (f.2) selecting the learning model that most precisely predicts and/or determines the relevant information, such as the relevant value and/or relevant value range of said parameter, for example. Alternatively, or in addition, a model can be selected that is capable of predicting the relevant information in advance at the earliest time during real time use is selected. Of course, other criteria for selecting a particular algorithm may apply.

Further regarding steps f.1) and f.2) above, exemplary models for establishing an algorithm for predicting and/or determining the relevant value or value range for said parameter may be selected from E/M (Expectation Maximization), NN (Neuronal Network) and HMM (Hidden Markov Models), and the method comprises comparing the performance of the different models for predicting the relevant value or value range in real time.

**Figure 7** illustrates an embodiment of a protocol and method steps conducted during the use of the surgical instrument in an operational environment. Step **61** in Fig. 7 is exemplary a step of screwing a screw or drilling into the bone of a patient. At step **62** an increment of the screw or drill bit insertion is made, and the torque is measured by the sensor **15.** The computer code in the data processing entity **20** (or **14, 16**) performs the algorithm at step **63,** which was established based on the experimental or learning data, which allows it to predict the optimal torque range (or far cortex) based on the incremental torque measurements. The process loops back at **66** to step **62,** where another incremental insertion of the screw (or drilling) is done. The looping and measuring continues until the measured torque is within the optimal torque range (or in case of drilling the far cortex is detected) at **64.** At this moment, the data processing entity produces an output at **65,** which may be the display of a warning on a screen and/or a remedial action, such as uncoupling the handle from the screwdriver shaft (or drill bit) so as to actively prevent further tightening.

Optionally, as shown by the dashed arrow leaving after step **64** and arriving at **67,** a feedback of the quality of the screw insertion (or drilling) may be sent back as an input to the algorithm. Another optional step is to perform a feed-forward of the drilling conditions to the algorithm at **68.**

In an embodiment, the invention provides the use of data corresponding to a particular patient and a particular operation for post-operative purposes. The recorded insertion torque evolution, for example, could be used for investigating the healing process for example, as part of an evidence-based medicine program. Data may also be useful as learning and clinical data for developers of other technology (such as orthopedic implant and instrument manufacturers. i.e. screw, screwdriver, drill bit, etc. designs) where historical operation data would be required. Such information may also be used for improving the algorithm used by the system of the invention.

A further advantage of embodiments of the electronic module described above is that since it can be embedded inside the handle of the surgical instrument, the complete instrument can be delivered as a sterile instrument ready for use. The entire instrument may be manufactured as a disposable instrument or on the other hand to be a reusable instrument.

The electronic module, assembly and/or the control system of the invention can be used for collecting data. The data may be collected for medicinal purposes, for example for post-operative study or review as well as data being delivered to the orthopaedic manufacturers for the future development of the implants and instruments or design changes where needed.

Accordingly, the data measured by said one or more sensor (e.g. tightening torque applied for fixing a bone screw) may be stored on a memory, for example on a database. For example, the data may be transmitted from the data processing entity 20, 120 to an external database. The data may be accompanied with data concerning the patent on which the data were collected, including data collected post-operatively from the patient. For example, the data may include data related to the stability over time of an implant (e.g. a bone screw or bone plate) fixed using the system and/or electronic module of the invention. Comparison of data collected during the surgical intervention with later data based on feedback of the patient or of the surgeon monitoring the patient can be used to improve the performance of the system of the invention, for example by improving the algorithms.

The data collected using the system, assembly and/or electronic module of the invention may be associated with data collected from a particular patient at a later stage, post-operatively. Such data may be, for example, a particular parameter value (e.g. maximum tightening torque value) that was used with a particular patient, and/or one or more landmark points that were detected or calculated during the surgical intervention. In the case of screw insertion, the screw specification may be stored and thus accessed post-operatively. Furthermore, if the tissue of the patient was categorized, such as bone quality, this information may also be collected and accessed later by an authorized person, such as a doctor, trained staff, and the like.

Accordingly, the collected data may be compared and/or correlated with medical parameters or performance indicators related to the implant, such as implant stability, success rate of the surgical intervention, but also more general parameters such as morbidity, mortality, healing outcome, and so forth.

While certain of the preferred embodiments of the present invention have been described and specifically exemplified above, it is not intended that the invention be limited to such embodiments. Various modifications may be made thereto without departing from the scope and spirit of the present invention, as set forth in the following claims.

## Claims

1. A control system (1) for a hand-held surgical instrument (5) comprising a handle (2) and an effector part (3), which is configured to act on a patient's tissue or on a piece to be connected to a patient's tissue, said the control system (1) comprising:
- an electronic module (10) comprising one or more sensor (15) configured to detect at least one parameter related to one or more physical properties or condition of a patient's tissue and to convert the detected parameter to an electrical signal, the electronic module (10) further comprising a battery (13) provided to supply electric energy to said sensor;
wherein said electronic module (10) is configured to be operably connected to a handle (2) and to an effector part (3) of said surgical instrument (5), the sensor (15) of the electronic module (10) being configured to connect to sense the parameter transferred to the sensor (15) via the effector part (3) when the surgical instrument (1) is used in an operational environment;
- a computer readable code configured to determine a relevant information that is relevant to a surgeon using the surgical instrument (5) in said operational environment,
wherein said computer readable code is further configured to perform an action when said relevant information is determined, wherein said action is selected from one or both of:
(a) induce the output of information via an output-unit (24), and
(b) acting on said surgical instrument (5) so as to affect the continued use of the surgical instrument (5) in said operational environment.

2. The control system (1) of any one of the preceding claims, wherein said electronic module (10) is configured to be replaceably and/or detachably interconnected between said handle (2) and said effector part (3).

3. The control system (1) of claim 1 or 2, comprising an assembly (25) comprising:
- said electronic module (10),
- a housing (17),
- first and second connectors (11, 12) for connecting said assembly (25) to said handle (2) and to said effector part (3) of said surgical instrument, respectively, and/or wherein said assembly (25) is removably and/or detachably connected to said surgical instrument, preferably between said handle and said effector part (3), and preferably outside said handle (2).

4. The control system (1) of any one of the preceding claims, comprising a data processing entity (20) configured to run said computer readable code to determine said relevant information, and to perform said action, such as the display of information on said output unit (24).

5. The control system (1) of any one of the preceding claims, wherein the electronic module (10) further comprises a transmitter (18) configured to wirelessly transmit and/or broadcast data and wherein said battery is provided to supply electric energy to said transmitter, wherein the data processing entity (20) comprises a receiver configured to receive the wirelessly transmitted signals transmitted by said transmitter (9).

6. The control system (1) of claim 4 and 5, wherein said data processing entity (20) is selected from the group consisting of a desktop computer, laptop computer, computer tablet and a smartphone.

7. The control system (1) of any one of the preceding claims, wherein said computer readable code is configured to determine said relevant information in real time.

8. The control system (1) of any one of the preceding claims, wherein said computer readable code comprises and/or makes use of an algorithm for determining said relevant information, wherein said algorithm configured to use successive and/or incremental electrical signals when the surgical instrument is used in an operational environment in order to determine one or more landmark points (30-33) or regions (35, 36) in a curve (C1, C2) based on said successive signals.

9. The control system (1) of claim 8, which is configured to determine said relevant information from said landmark points (31-33) and/or landmark regions (35, 36) and/or from an extrapolated curve determined from said landmark points and/or landmark regions.

10. The control system (1) according to any one of the preceding claims, wherein said computer readable code comprises instructions to create one or more auxiliary line and/or function (L1-L3), and determining an intersection of said auxiliary line and/or function with a curve (C1) representing the evolution of the parameter as detected by said sensor, wherein said intersection constitutes said relevant information.

11. The control system (1) of claim 10, wherein said curve (C1) is an at least partially extrapolated curve, created by said computer readable code on the basis of landmark points (31-33) and/or landmark regions (35, 36) detected by the system in real time during an on-going surgical intervention, and wherein said intersection is determined by calculation and/or extrapolation.

12. The control system (1) according to any one of claim 10 and claim 11, wherein said curve (C1) comprises a linear region (36), and wherein said auxiliary line (L1-L3) is positioned so as to be in parallel to said linear region or at an angle with respect to said linear region.

13. The control system (1) according to any one of the preceding claims, wherein said relevant information is selected from the group consisting of:
(a) the detection of the occurrence of a relevant event during an on-going surgical intervention,
(b) the determination of a relevant value or relevant value range, in particular with respect to said parameter.

14. The control system (1) according to claim 13, wherein said relevant value or relevant value range is determined in advance, in particular before said relevant value or relevant value range is reached during an on-going surgical intervention in which said surgical tool (5) being is used.

15. The control system (1) of claim 13 or 14, wherein said relevant event is selected from the group consisting of:
(i) the detection of a situation where, during a surgical intervention, a relevant value and/or relevant value range of said parameter has been reached;
(ii) the detection of a contact of a head (4) of the effector part (3) or of a tip of said a piece to be connected to a patient's tissue with tissue having different properties and/or a different structure.

16. The control system (1) according to any one of the preceding claims, wherein said relevant information is the detection of a contact of a head (4) of the effector part (3) or of the tip of said piece to be connected to a patient's tissue with a second or far cortex.

17. The control system (1) according to any one of the preceding claims, wherein said computer readable code comprises and/or makes use of an algorithm configured to attribute, in real time, a given tissue upon which said surgical instruments (1) acts, to a particular category out of a plurality of categories, and wherein a relevant value or relevant value range is associated with a particular category, such that said relevant value or optimal value range ensues directly from said category.

18. An electronic module (10, 10') comprising one or more sensor (15) configured to detect at least one parameter related to one or more physical properties or condition of a patient's tissue and to convert the detected parameter to an electrical signal, the electronic module (10) further comprising a battery (13) provided to supply electric energy to said sensor.

19. The electronic module (10, 10') of claim 18, which is configured to be replaceably and/or detachably interconnected between a handle (2) and an effector part (3) of a surgical instrument.

20. The electronic module of claim 18, which is configured to be placed in the handle (2) of a surgical instrument.

21. The electronic module of any one of claims 18-20, which further comprises a transmitter (18) configured to wirelessly transmit and/or broadcast data and wherein said battery is provided to supply electric energy to said transmitter.

22. An assembly (25, 125) comprising:
- an electronic module (10, 10') comprising one or more sensor (15) configured to detect at least one parameter related to one or more physical properties or condition of a patient's tissue and to convert the detected parameter to an electrical signal, the electronic module (10) further comprising a battery (13) provided to supply electric energy to said sensor,
- a housing (17),
- first and second connectors (11, 12) for connecting said assembly (25) to said handle (2) and to said effector part (3) of said surgical instrument, respectively, and/or wherein said assembly (25) is removably and/or detachably connected to said surgical instrument, preferably between said handle and said effector part (3), and preferably outside said handle (2).

23. A module preferably connectable to the conventional orthopedic instruments such as screwdrivers and power tools which can be collecting the data (physical properties or condition of a patient's tissue) for a postoperative studies and evidences.

24. A method of collecting data, the method comprising storing on a database the data generated by the one or more sensors of the electronic module (10, 10') of claim 18 when using a surgical instrument (5, 105) comprising the electronic module (10, 10'), preferably together with data related to the patient on which the surgical instrument (5, 105) was used.
